# EUROPEAN PATENT APPLICATION

(11) **EP 2 559 385 A2**
(43) Date of publication of application: **20.02.2013**
(21) Application number: 11769135.2
(22) Date of filing: 18.04.2011
(51) Int. Cl.: A61B 17/02, A61B 1/24, A61B 17/24, A61C 19/00

(54) **RETRACTOR FOR DENTAL TREATMENT**

(30) Priority: 16.04.2010 KR 20100035071; 15.04.2011 KR 20110035098
(71) Applicant: B&L Biotech Co., Ltd., 1136-1 Sanbon-dong Gunpo-si, Gyeonggi-do 435-040 (KR)
(72) Inventor: LEE, In Hwan, Seoul 135-110 (KR)
(74) Representative: Tischner, Oliver
(86) International application number: PCT/KR2011/002748
(87) International publication number: WO 2011/129669

(57) **Abstract**

A dental retractor made of a shape memory material to be easily bended and restored to an initial shape under predetermined conditions is provided. The dental retractor includes a retraction portion made of nitinol or alloy including nitinol to be bendable and to have shape memory characteristics, and configured to hold gingival tissue by being supported by a tooth; a support portion connected to one side of the retraction portion to support the retraction portion; and a handle portion connected to the support part. Since the dental retractor is easily bent and variously deformed as necessary, convenience of a surgery may be increased. In addition, since the dental retractor is restored to the initial shape under the predetermined conditions after the surgery, convenience is increased.

## Description

### Technical Field

The present invention relates to a dental retractor, and more particularly, to a dental retractor including a shape memory material to be easily bent as necessary and restored to an initial shape under predetermined conditions, and efficiently fixing tissue of a wide area.

### Background Art

Generally, when performing an implant surgery or other dental surgeries, a mount of a patient is opened wide by pulling back lips of upper and lower jaws to secure an adequate field of view.

When gingival tissue covering teeth is cut to treat dental root and the like during a treatment with the mouth of the patient opened, if the gingival tissue is not securely held, the treatment may be seriously affected. Therefore, in various dental treatments or operations, a dental retractor is used to fix the gingival tissue covering the teeth and to secure a field of view within an oral cavity of the patient, thereby achieving a convenient operation.

A general dental retractor is structured such that a metal wire having a predetermined thickness forms a frame. For example, the general dental retractor may include a handle portion and a retraction portion that holds gingival tissue.

Since the general dental retractor is made of general metal, deformation of the dental retractor is difficult. Therefore, once worn on an open mouth of a patient, the dental retractor is hard to move, thereby causing a spatial limit during a surgery.

In addition, the general dental retractor is limited in size of a front end so that the gingival tissue only in a size covering one tooth may be fixed. Therefore, when a relatively wide area of gingival tissue covering a plurality of teeth is to be fixed, use of the dental retractor is limited.

Accordingly, there is a desire for a new dental retractor capable of supporting a wide area of gingival tissue simultaneously during a surgery of a plurality of teeth or gingiva covering a plurality of teeth while securing an adequate field of view and minimizing a spatial limit, and also capable of being flexibly bent.

### Disclosure of Invention

### Technical Goals

An aspect of the present invention provides a dental retractor made of a shape memory material which is easily bent and restored to an initial shape under predetermined conditions, so as to minimize a spatial limit and secure a field of view during a dental surgery, thereby increasing convenience of the dental surgery.

Another aspect of the present invention provides a dental retractor applicable to various uses of a dental surgery according to shapes of a tooth and gingival tissue or a shape of gingival tissue according to contact relations between teeth.

Still another aspect of the present invention provides a dental retractor for supporting a relatively wide area of gingival tissue during a surgery of a plurality of teeth or gingiva covering a plurality of teeth.

### Technical solutions

According to an aspect of the present invention, there is provided a dental retractor including a retraction portion made of nitinol or alloy including nitinol to be bendable and to have shape memory characteristics, and configured to hold gingival tissue by being supported by a tooth; a support portion connected to one side of the retraction portion to support the retraction portion; and a handle portion connected to the support portion.

The retraction portion may include a wing portion protruding at each of opposite sides; and a support part protruding at a front part and including a serrated portion that includes a plurality of teeth at the front part, wherein the retraction portion may be entirely in a cross shape. The wing portion may protrude to the opposite sides by the same length or different lengths. The wing portion may include a rounded portion formed at an end. The wing portion may include a sub serrated portion formed in at least one position of a front part of the wing portion. An end of the support part, in which the serrated portion is formed, may protrude from the retraction portion to be parallel or inclined. The serrated portion may include a plurality of saw teeth in any one shape or at least two shapes selected from a triangle and other polygons.

The retraction portion may be made of alloy of nitinol and a bendable material including steel use stainless (SUS).

A side surface of the support portion to contact lips of a patient during a surgery may be formed as a curved surface.

The dental retractor may further include an expansion portion connected at a connection part between the support portion and the retraction portion to expand a volume of the retraction portion and the support portion.

The expansion portion may be made of a soft and elastic material including silicone.

The support portion and the handle portion may be separately formed to be fastened, and the support portion may include a screw thread at an end so that the support portion and the handle portion are screw-connected to each other.

### Effects of Invention

According to embodiments of the present invention, a dental retractor is made of a shape memory material which is easily bent as necessary and restored under predetermined conditions after a surgery. Thus, since the dental retractor is freely bent, a spatial limit may be reduced while a field of view is secured. As a result, convenient of the surgery may be increased.

Additionally, according to embodiments of the present invention, the dental retractor may be used in various angles and shapes by being bent according to shapes of a tooth and gingival tissue or a shape of gingival tissue according to contact relations between teeth. Accordingly, efficiency of the surgery may be increased.

Additionally, according to embodiments of the present invention, during a surgery of a plurality of teeth or gingiva covering a plurality of teeth, since a relatively wide area of gingival tissue covering the plurality of teeth may be supported simultaneously, the surgery may be conveniently performed.

### Brief Description of Drawings

FIG. 1 is a diagram illustrating a dental retractor according to an embodiment of the present invention;
FIG. 2 is a diagram illustrating a thickness of the dental retractor shown in FIG. 1;
FIG. 3 is a diagram illustrating a shape of one side of a support part to contact a bone structure, according to the embodiment of the present invention;
FIG. 4 is a diagram illustrating a second bending recess, according to the embodiment of the present invention;

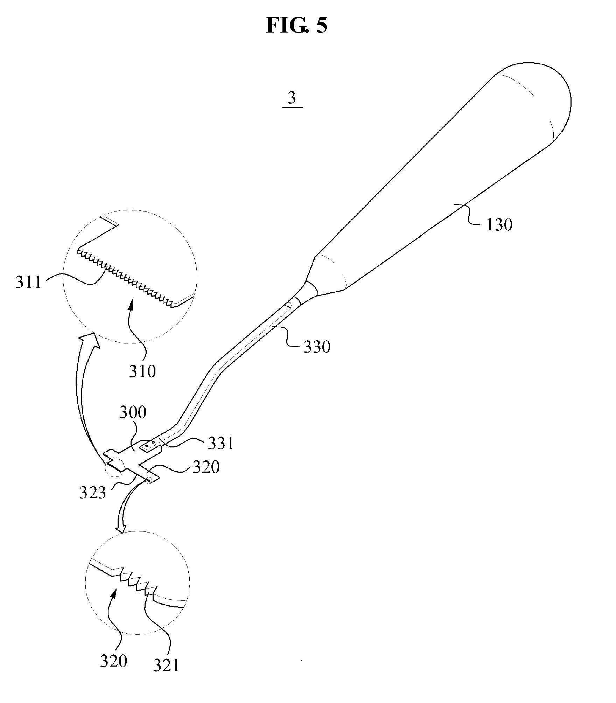

FIG. 5 is a perspective view illustrating a dental retractor according to another embodiment of the present invention;

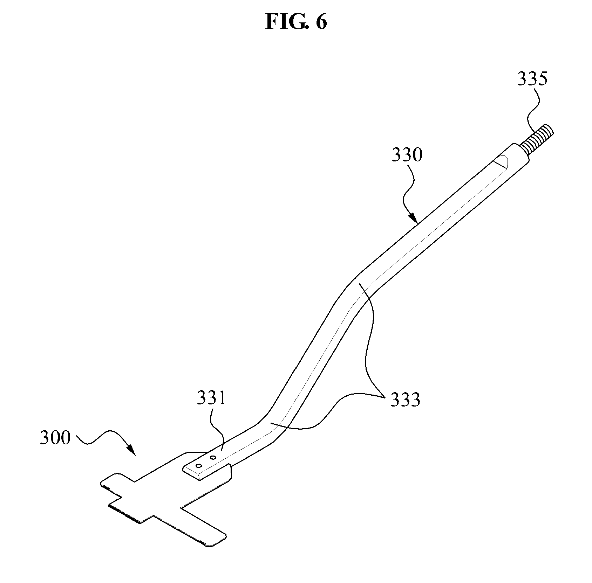

FIG. 6 is a plan view illustrating main parts of the dental retractor of FIG. 5;

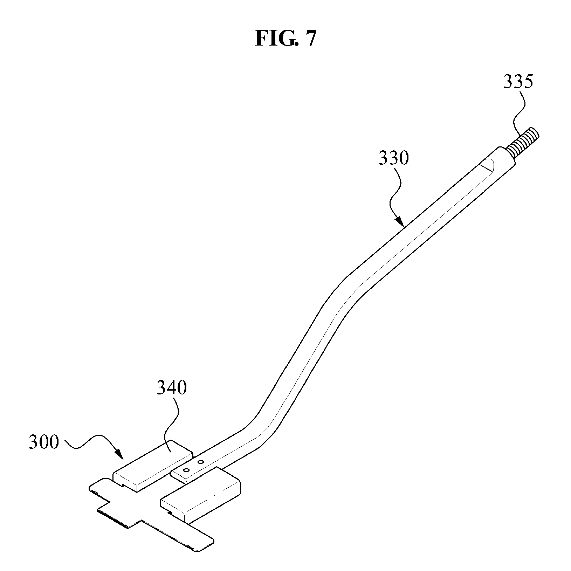

FIG. 7 is a plan view illustrating a dental retractor modified from the dental retractor of FIG. 6;
FIG. 8 is a diagram illustrating operation of a retraction portion of the dental retractor of FIG. 5; and
FIGS. 9 to 16 are plan views illustrating a shape of a retraction portion according to modified embodiments of the present invention.

### Best Mode for Carrying Out the Invention

Reference will now be made in detail to exemplary embodiments of the present invention, examples of which are illustrated in the accompanying drawings, wherein like reference numerals refer to the like elements throughout. Well-known functions or structures will not be described in detail not to obscure subject matters of the present invention.

A dental retractor 1 according to an embodiment of the present invention will be described as a surgical retractor generally used in a dental surgery. The dental retractor 1 may be disposed at a necessary position for the dental surgery to hold tissue. Usually, the tissue may refer to gingival tissue although not limited. The dental retractor 1 may be employed for various uses as far as tissue needs to be fixed in various dental surgeries.

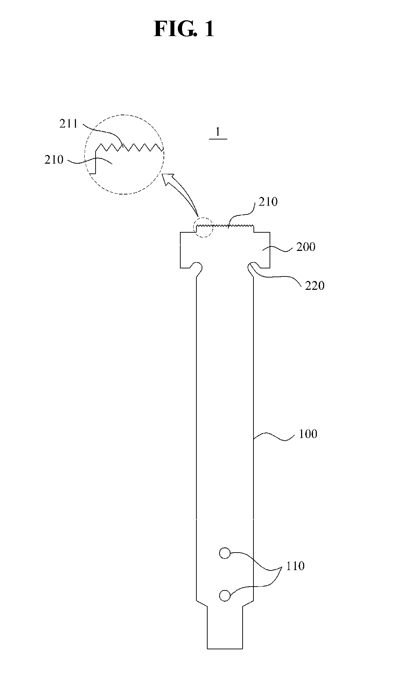

The dental retractor 1 may include a handle portion 100 and a retraction portion 200. The structure of the dental retractor 1 will be described in further detail with reference to FIG. 1.

FIG. 1 is a diagram illustrating the dental retractor 1 according to the embodiment of the present invention.

The handle portion 100 is extended into a predetermined length to function as a handle for holding of the dental retractor 1 during a surgery. The retraction portion 200 may be formed at one end of the handle portion 100 to fix skin tissue cut during the surgery. A connection hole 110 may be formed at an opposite end of the handle portion 100. Another handle or other parts may be connected through the connection hole 110.

One side of the retraction portion 200 may be connected to one end of the body portion while an opposite side of the retraction portion 200 may be connected to a tooth. The retraction portion 200 is adapted to retract the skin tissue during the surgery. In detail, an end of the retraction portion 200 is supported by a bone structure such as the tooth. In a case where tissue surrounding a tooth is cut, the retraction portion 200 holds the cut tissue from covering the tooth again.

In addition, the retraction portion 200 may not be immovably connected to the handle portion 100. That is, the retraction portion 200 and the handle portion 100 may move relative to each other. Therefore, bending of the retraction portion 200 is available as will be described later.

The handle portion 100 and the retraction portion 200 may be integrally formed with each other but not limited thereto.

The handle portion 100 and the retraction portion 200 may be made of a material including nitinol to be easily bent by a human force and to have shape memory characteristics. However, various other materials having the shape memory characteristics may be alterably used. Nitinol refers to non-magnetic alloy containing nickel and titanium almost half and half. Though deformed by an external operation such as bending and distorting, nitinol restores to an initial shape by heating to a predetermined temperature or dipping in water due to the shape memory characteristics.

According to an exemplary embodiment, nitinol restoring to the initial shape at about 50° or higher may be used. Since the handle portion 100 and the retraction portion 200 are made of nitinol which is shape memory alloy, a doctor using the dental retractor 1 may properly bend only the handle portion 100 or the retraction portion 200, thereby securing a space and a field of view. Thus, convenience of the surgery may be increased. Furthermore, since the dental retractor 1 may restore to the initial shape under a predetermined temperature, the dental retractor 1 may be easy to reuse after the surgery.

The retraction portion 200 may include a support part 210. The support part 210 may be protruded from an end of the retraction portion 200. The support part 210 may support the dental retractor 1 in contact with the bone structure such as the tooth during the surgery. A predetermined recess may be formed at the bone structure for insertion of the support part 210 in the bone structure in a contacting manner. Alternatively, the support part 210 may directly contact an outer surface of the bone structure.

Opposite sides of the retraction portion 200, excluding the end where the support part 210 is protruded and the end connected to the handle portion 100, may be extended into a bar shape. That is, on the same plane as the handle portion 100, the opposite sides of the retraction portion 200 are extended orthogonal to a length direction of the handle portion 100. Due to the foregoing structure of the retraction portion 200, the dental retractor 1 may have a cross shape as a whole. Therefore, when the surgery is performed with respect to a plurality of teeth or corresponding gingival, the extended part of the retraction portion 200 may support a wide area of gingival tissue or skin tissue simultaneously.

However, when supporting of a wide area of tissue is unnecessary, only the extended part of the retraction portion 200 may be bent and used since the retraction portion 200 is made of nitinol. Thus, various areas of tissue may be supported.

A first bending recess 220 may be recessed inwardly from opposite ends of a connection part between the retraction portion 200 and the handle portion 100. The first bending recess 220 may be provided in various shapes. The presence of the first bending recess 220 may enable the retraction portion 200 to be more easily bent with respect to the handle portion 100. Therefore, the doctor may easily secure the space and the field of view for the surgery, by bending up and down the handle portion 100 of the dental retractor 1 linearly protruding from an open mouth of the patient.

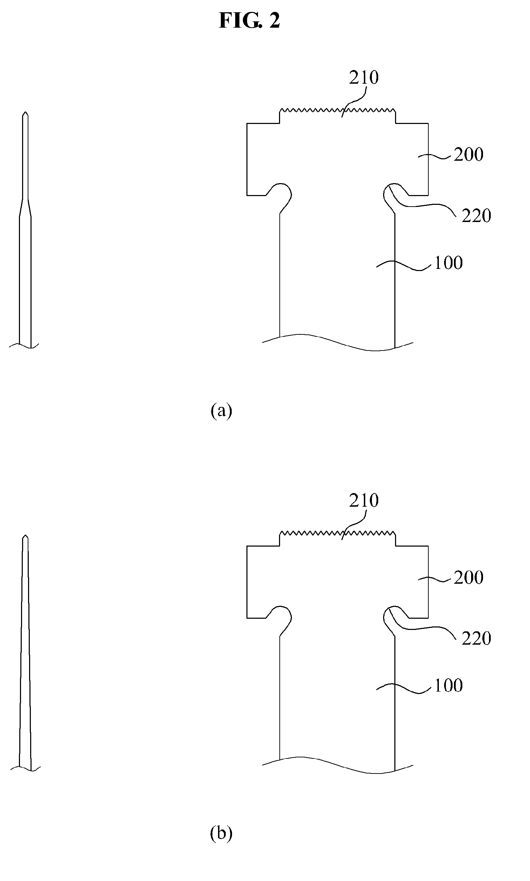

As shown in FIG. 2, the connection part between the retraction portion 200 and the handle portion 100 may be thinner than the handle portion 100 so that the retraction portion 200 may more easily bend with respect to the handle portion 100.

To be more specific, as shown in (a) of FIG. 2, a thickness may reduce from the connection part between the retraction portion 200 and the handle portion 100 such that an entire thickness of the retraction portion 200 becomes smaller than a thickness of the handle portion 100. Also, as shown in (b) of FIG. 2, a thickness of another side of the handle portion 100, not connected with the retraction portion 200, may gradually reduce toward the support part 210. Therefore, the retraction portion 200 that holds the cut tissue by being supported by the bone structure may be easily bent with respect to the handle portion 100. As a result, convenience and efficiency of the surgery may increase.

One side of the support part 210, which meets the tooth, may include a serrated portion 211. The serrated portion 211 may secure support of the support part 210 by the bone structure such as the tooth when contacting the bone structure. The serrated portion 211 may have a triangle shape. However, not specifically limited, the serrated portion 211 may be in any of various shapes functioning as a protrusion.

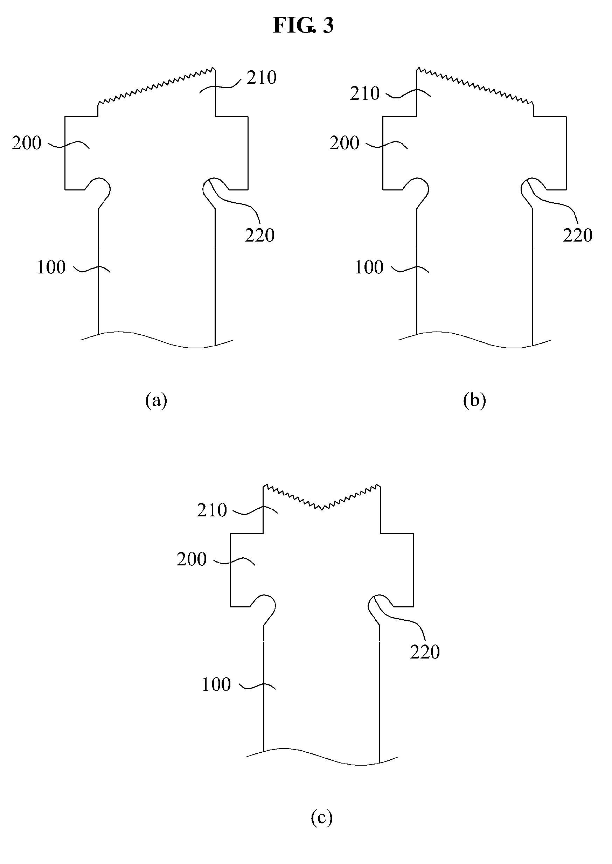

One side of the support part 210, which contacts the bone structure, may be changed in shape as necessary. This will be described in detail with reference to FIG. 3. FIG. 3 is a diagram illustrating the shape of one side of the support part 210 to contact the bone structure, according to the embodiment of the present invention.

As shown in (a) and (b) of FIG. 3, the support part 210 may be inclined such that a length protruding from the retraction portion 200 is not constant. Therefore, a proper shape of the support part 210 may be employed according to the shape of the bone structure, accordingly easily securing the space and the field of view during the surgery.

Alternatively, the support part 210 may have an M shape in which both ends are more protruded than a middle portion as shown in (c) of FIG. 3.

The support part 210 in such various shapes may be integrally formed with the retraction portion 200. However, as necessary, the support part 210 may be removably connected.

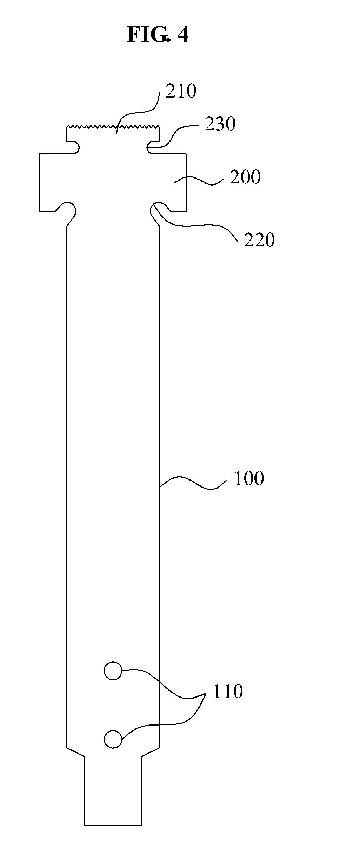

According to the embodiment, as shown in FIG. 4, a second bending recess 230 may be formed inwardly at opposite sides of a portion at which the support part 210 protrude from the retraction portion 200. By such a structure, the retraction portion 200 may more easily bend with respect to the support part 210.

In the dental retractor 1 according to the foregoing embodiments in which the handle portion 100 and the retraction portion 200 are integrally formed, nitinol is applied only to the retraction portion 200 to be bendable. That is, the retraction portion 200 and the handle portion 100 may be separately manufactured and then connected. The retraction portion 200 may be made of nitinol to be freely bent and restored to an initial shape after use whereas the handle portion 100 is made of a high-strength material for a convenient use.

Hereinafter, a dental retractor 3 configured in such a manner that a retraction portion is separately manufactured and connected will be described. In the following description, elements substantially the same as in the dental retractor 1 of the previous embodiments will be cited by the same reference numerals.

The dental retractor 3 may include a retraction portion 300, a support portion 330, and a handle portion 130.

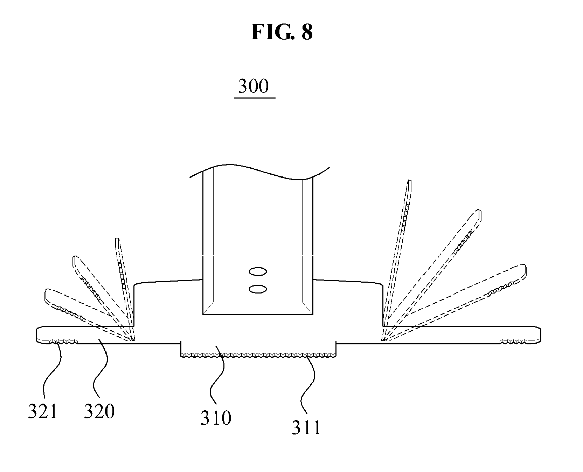

The retraction portion 300 is easy to bend even by a human force. The retraction portion 300 may be made of a material including nitinol which has shape memory characteristics to be restored to an initial shape under predetermined conditions, for example when heated. The retraction portion 300 may be made of alloy including steel use stainless (SUS) and nitinol so as to be easily deformed. As shown in FIG. 8, the retraction portion 300 may be freely bent. Therefore, the retraction portion 300 may be used by being deformed corresponding to a shape of a tooth undergoing the surgery.

The support portion 330 may support the retraction portion 300. The support portion 330 may be made of any of various materials including metal, alloy, and resin having predetermined strength. The retraction portion 300 and the support portion 330 may be integrally formed with each other.

When the support portion 330 is mounted in the mouth of the patient undergoing the surgery, a side of the support portion 330 may touch lips. To prevent an injury or inconvenience of the lips in this case, the side of the support portion 330 may be curved. For example, the support portion 330 may be provided in a bar shape having a semicircular cross section. In addition, to increase convenience of the surgery, bending portions 332 and 333 may be formed in at least one position of the support portion 330. The position, a number, and a bending angle of the bending portions 332 may be varied.

According to the present embodiment, the dental retractor 3 may include a fastening portion 335 formed at an end of the support portion 330, which is not connected to the retraction portion 300, for fastening of the handle portion 130. Therefore, various shapes of the retraction portion 300 may be applied. For example, the support portion 330 and the handle portion 130 may each include a screw thread at an end for screw-connection. The support portion 330 may be inserted in the handle portion 130 and screw-connected. However, the present invention is not limited to the drawings but various fastening structures between the support portion 330 and the handle portion 130 may be applied.

The handle portion 130 may be shaped to be conveniently grabbed by an operator during the surgery, and may have a general handle shape. Various materials such as metal and resin may be applied to the handle portion 130. Since various types of the retraction portion 300 may be applied to one handle portion 130, convenience in storage and use may be increased.

In addition, the dental retractor 3 may include an expansion portion 340 connected to a connection part between the support portion 330 and the retraction portion 300. The expansion portion 340 may expand a volume of the retraction portion 300 and the support portion 330, thereby securing a space in the mouth. For example, the expansion portion 340 may have a substantially flattened-U-shape in which the support portion 330 and the retraction portion 300 surround a connection portion 331 at opposite lateral sides. To secure the space, the expansion portion 340 may be formed thicker than the support portion 330 and the retraction portion 300. Here, the expansion portion 340 may be made of a soft and elastic material to prevent a part of the expansion portion 340 contacting the mouth from causing an injury or inconvenience.

To describe a structure of the retraction portion 300, a serrated portion 311 including a plurality of saw teeth at a leading end may protrude from a front part of a support part 310 and, additionally, a wing portion 320 may protrude from both sides. Accordingly, the retraction portion 300 may entirely have a substantial cross shape.

The support part 310 may include the serrated portion 311 for abrasion at a front part to contact the tooth. The serrated portion 311 may linearly protrude parallel with the retraction portion 300. However, the serrated portion 311 may be inclined with respect to the retraction portion 300. Alternatively, as in the previous embodiment, the serrated portion 311 may have an M shape in which both ends protrude more than a middle part.

Although the serrated portion 311 is shown to have triangular saw teeth, the present invention is not limited to the drawing. The saw teeth may have any of various shapes. In addition, the serrated portion 311 may have one shape saw teeth or combination of various shapes may be applied. In the drawing of the present embodiment, the size and the shape of the serrated portion 311 are exaggerated. That is, the serrated portion 311 may actually have a micro size as far as the serrated portion 311 prevents slip of the dental retractor 3 by providing abrasion against the tooth.

The wing portion 320 may protrude from both sides of the retraction portion 300. An end of the wing portion 320 may be rounded to prevent an injury or inconvenience to the mouth. In addition, a sub serrated portion 321 including a plurality of micro teeth may be formed in at least one position of the rounded end for stable support. For example, as shown in the drawing, the sub serrated portion 321 may be formed at a part of a front rounded end of the wing portion 320. Here, a size, a position, and a number of the micro teeth of the sub serrated portion 321 may be varied. The sub serrated portion 321 may even be omitted.

The protruding parts of the wing portions 320 protruding from the left and the right may have the same length or different lengths.

In the drawing, a reference numeral 323 refers to a leading end of the wing portion 320.

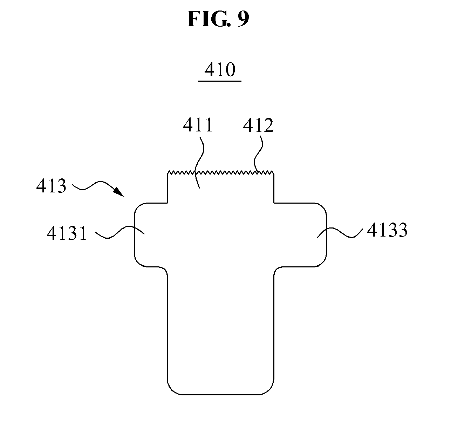

More detailed shapes of the retraction portion are described in FIGS. 9 through 16. In the following description, the retraction portions are actually the same except for the shape. For a convenient explanation, the description will be made briefly only about the shape.

The dental retractor 3 may have various shapes of the retraction portion. The retraction portion may have various shapes of a support portion and a wing portion.

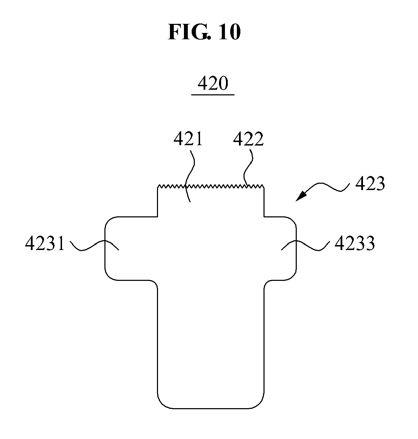

The dental retractors shown in FIGS. 9 and 10 respectively include retraction portions 410 and 420 extended in a constant thickness up to support parts 411 and 421. The retraction portions 410 and 420 may include wing portions 413 and 423 extending to opposite lateral sides from positions adjacent to the support parts 411 and 421 disposed at upper ends, thereby forming a substantial cross shape. Serrated portions 412 and 422 of the support parts 411 and 421 may be linear. However, the retraction portion 410 of FIG. 9 has a right wing portion 4133 protruding longer than a left wing portion 4131. The retraction portion 420 of FIG. 10 has a left wing portion 4231 protruding longer than a right wing portion 4233.

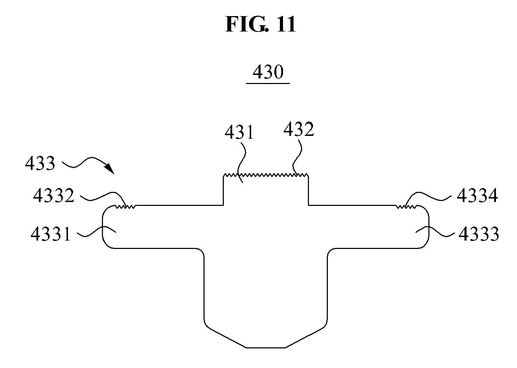

In a retraction portion 430 shown in FIG. 11, a support part 431 is narrower than an entire body of the retraction portion 430, and a serrated portion 432 protrudes in parallel. A wing portion 433 may include a left wing portion 4331 and a right wing portion 4333 having the same length. Sub serrated portions 4332 and 4334 may be formed at part of a front end of the wing portion 433.

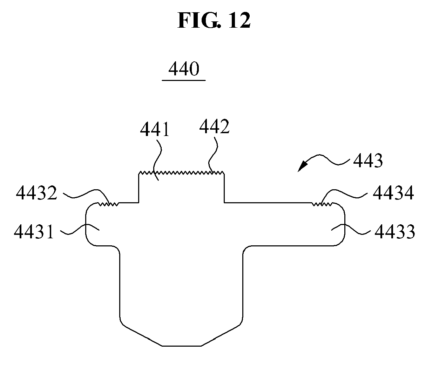

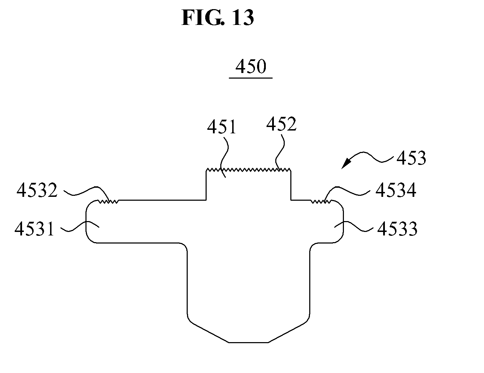

In a similar manner to the retraction portion 430 of FIG. 11, FIGS. 12 and 13 respectively show support parts 441 and 451 protruding to be narrower than an entire body, and serrated portions 442 and 452 formed at ends of the support parts 441 and 451. In addition, sub serrated portions 4432, 4434, 4532, and 4534 are formed at part of front ends of wing portions 440 and 450. However, different from the embodiment of FIG. 11, retraction portions 440 and 450 according to the embodiments of FIGS. 12 and 13 may include wing portions 443 and 453 of which left and right wing portions have different lengths. The retraction portion 440 of FIG. 12 includes a right wing portion 4433 protruding longer than a left wing portion 4431. The retraction portion 450 of FIG. 10 includes a left wing portion 4531 protruding longer than a right wing portion 4533.

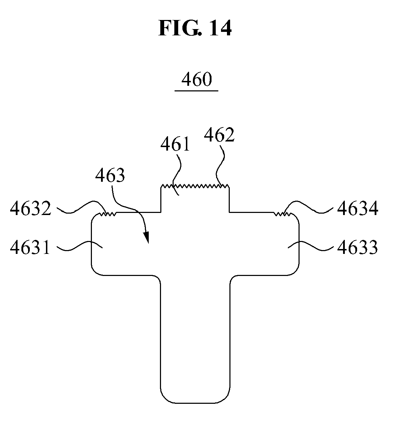

Retraction portions 460, 470, and 480 shown in FIGS. 14 to 17 may have a substantial cross shape. Compared to in the retraction portions 410 and 420 shown in FIGS. 1 and 2, wing portions 463, 473, and 483 of the retraction portions 460, 470, and 480 may be extended longer.

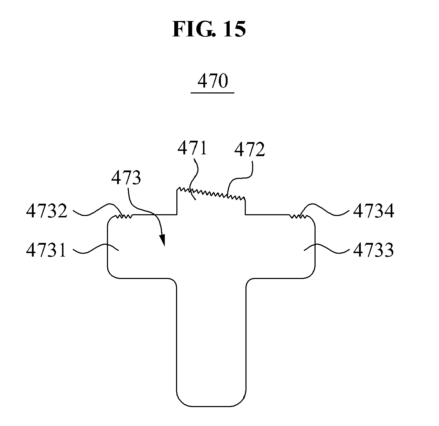

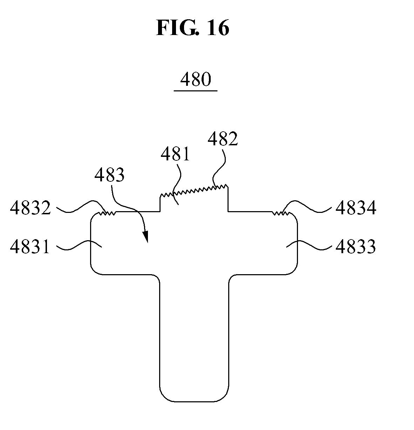

The retraction portion 460 of FIG. 14 may include a support part 461 formed in parallel. A support part 471 of the retraction portion 470 of FIG. 15 may be inclined downward toward the right. A support part 481 of the retraction portion 480 of FIG. 16 may be inclined downward toward the left.

In FIGS. 14 to 16, non-described reference numerals 463, 4631, 4633, 473, 4731, 4733, 483, 4831, and 4833 denote left wing portions and right wing portions of the retraction portions 460, 470, and 480, respectively. Also, non-described reference numerals 4632, 4634, 4732, 4734, 4832, and 4834 denote sub serrated portions.

Hereinafter, the operation of the dental retractors 1 and 3 structured as described above according to the embodiments of the present invention will be described.

When various surgeries are performed in a dental clinic, a patient opens his or her mouth. In this state, a tooth or gingival tissue to undergo the surgery is cut. The cut tissue needs to be fixed in a retracted state during the surgery so that the surgery is conveniently performed and a field of view of a doctor is secured.

After the tissue is cut, the tissue is hold up or down. In this state, a bone structure, for example the tooth, is brought into contact with one side of a support part that includes a serrated portion. Next, when a handle portion is held and lifted, the dental retractors 1 and 3 made of nitinol or alloy including nitinol and SUS may be bent up or down with the support part fixed to the tooth, and maintain this state. Here, the tissue retracted by a retraction portion is fixed. Bending of the dental retractors 1 and 3 may be achieved in various types depending on various shapes and thicknesses of the retraction portion and a wing portion. Also, partial and entire bending may both be possible. Since the dental retractors 1 and 3 are freely deformed, the field of view and the space for the surgery may be easily secured, accordingly increasing convenience in the surgery.

After the surgery, the dental retractors 1 and 3 are removed from the tooth and the tissue is restored to an initial position and sutured. Since the dental retractors 1 and 3 are made of nitinol or alloy including nitinol and SUS, the dental retractors 1 and 3 may be restored to the initial shape under predetermined temperature conditions to be reused for a next surgery.

Although a few embodiments of the present invention have been shown and described, the present invention is not limited to the described embodiments. Instead, it would be appreciated by those skilled in the art that changes may be made to these embodiments without departing from the principles and spirit of the invention, the scope of which is defined by the claims and their equivalents.

## Claims

1. A dental retractor comprising:
a retraction portion made of nitinol or alloy including nitinol to be bendable and to have shape memory characteristics, and configured to hold gingival tissue by being supported by a tooth;
a support portion connected to one side of the retraction portion to support the retraction portion; and
a handle portion connected to the support portion.

2. The dental retractor of claim 1, wherein the retraction portion comprises :
a wing portion protruding at each of opposite sides; and
a support part protruding at a front part and including a serrated portion that includes a plurality of teeth at the front part,
wherein the retraction portion is entirely in a cross shape.

3. The dental retractor of claim 2, wherein the wing portion protrudes to the opposite sides by the same length or different lengths.

4. The dental retractor of claim 2, wherein the wing portion comprises a rounded portion formed at an end.

5. The dental retractor of claim 2, wherein the wing portion comprises a sub serrated portion formed in at least one position of a front part of the wing portion.

6. The dental retractor of claim 2, wherein an end of the support part, in which the serrated portion is formed, protrudes from the retraction portion to be parallel or inclined.

7. The dental retractor of claim 2, wherein the serrated portion comprises a plurality of saw teeth in any one shape or at least two shapes selected from a triangle and other polygons.

8. The dental retractor of claim 1, wherein the retraction portion is made of alloy of nitinol and a bendable material including steel use stainless (SUS).

9. The dental retractor of claim 1, wherein a side surface of the support portion to contact lips of a patient during a surgery is formed as a curved surface.

10. The dental retractor of claim 1, further comprising an expansion portion connected at a connection part between the support portion and the retraction portion to expand a volume of the retraction portion and the support portion.

11. The dental retractor of claim 10, wherein the expansion portion is made of a soft and elastic material including silicone.

12. The dental retractor of claim 1, wherein
the support portion and the handle portion are separately formed to be fastened, and
the support portion comprises a screw thread at an end so that the support portion and the handle portion are screw-connected to each other.
